(19) Europäisches Patentamt
European Patent Office
Office européen des brevets

(11) **EP 1 082 051 B1**

(12) **EUROPEAN PATENT SPECIFICATION**

(45) Date of publication and mention
of the grant of the patent:
**03.01.2007 Bulletin 2007/01**

(21) Application number: **99926043.3**

(22) Date of filing: **28.05.1999**

(51) Int Cl.:
***A61B 5/00*** (2006.01)

(86) International application number:
**PCT/US1999/011973**

(87) International publication number:
**WO 1999/060922 (02.12.1999 Gazette 1999/48)**

(54) **FREQUENCY ESTIMATION OF ELECTRO-ISLET-GRAPHY**

FREQUENZSCHÄTZUNG BEI ELEKTRO-ISLET-GRAPHIE

ESTIMATION DE FREQUENCES EN ELECTRO-INSULO-GRAPHIE

(84) Designated Contracting States:
**AT BE CH CY DE DK ES FI FR GB GR IE IT LI LU MC NL PT SE**

(30) Priority: **28.05.1998 US 87026 P**

(43) Date of publication of application:
**14.03.2001 Bulletin 2001/11**

(73) Proprietor: **Carmel Biosensors Ltd.**
**Haifa 31905 (IL)**

(72) Inventor: **CHARASH, Dan**
**31905 Haifa (IL)**

(74) Representative: **Charlton, Peter John**
**Elkington and Fife LLP,**
**Prospect House**
**8 Pembroke Road**
**Sevenoaks,**
**Kent TN13 1XR (GB)**

(56) References cited:
**US-A- 5 101 814**

- **SIMMONS A M, FERRAGAMO M: "Periodicity extraction in the anuran auditory nerve. I. "Pitch-shift" effects" JOURNAL OF COMPARATIVE PHYSIOLOGY. A, SENSORY, NEURAL, AND BEHAVIORAL PHYSIOLOGY, vol. 172, no. 1, February 1993 (1993-02), pages 57-69, XP008026771 ISSN: 0340-7594**

**Description**

<u>Field of the Invention</u>

The present invention relates generally to methods and systems for processing electro-physiological signals and, more particularly, concerns the detection and analysis of electrical activity related to blood glucose concentration.

<u>Background of the Invention</u>

Blood glucose level monitoring is of great importance for diabetics. Continuous monitoring of the glucose level can greatly reduce the medical complications, that are caused by metabolic imbalance.

The Islets of Langerhans are located in the pancreas, and are responsible for the manufacture of insulin in the human body. An islet is a cluster of many cells. The Beta cells within the islets respond to glucose in bursts of electrical activity. The use of such islets, derived from the pancreas of donor animals, in a blood glucose monitor is disclosed in U.S. Patent 5,101,814. Electro-Islet-Graphy (EIG) is the measurement of the electrical activity of the islets of Langerhans. The present invention utilizes EIG to provide a continuous blood glucose level sensor.

Studies demonstrate a clear correlation between the fundamental frequency of the EIG signal, and the glucose level in the medium surrounding the islet. Hence, the estimation of the fundamental frequency of Electro-Islet-Graphy is of significant practical value.

The fundamental frequency is defined as the frequency of the "events" of the EIG. An "event" is believed to represent the synchronized electrical activity of the cells in the islet. By analogy to an ECG, an "event" in EIG is comparable to a the heart cycle (the PQRST comlex).

Although it is believed that EIG processing has not been performed by any entity but the owner the present patent application, one might attempt to detect the events directly, and then to calculate the fundamental frequency. The problem with this approach is that the shape and size of EIG "events" varies greatly, and therefore reliable and robust event detection is difficult to achieve.

In accordance with the present invention, the fundamental frequency of EIG events is determined directly, without first detecting the individual events themselves. All these algorithms must use an analysis window containing more than one event. The present invention utilizes techniques similar to those utilized to detect and estimate pitch in speech processing

The invention is based on two important biological discoveries. The first is that the EIG is generated by a functional pace maker. The second is that the EIG signal is *quasi-periodic* most of the time. Pitch detection algorithms are used, because of the essentially quasi-periodic nature of the EIG signal. By *quasi-periodic* we mean that (1) the intervals between successive events are not exactly identical, but may vary slightly and (2) the amplitude and shape of successive events may also exhibit some variance.

Several pitch detection algorithms were tested. Three of them achieved good results: Autocorrelation, Segmented Autocorrelation and Harmonic Peaks analysis. The preferred embodiments of the invention focus on algorithms that are based on the Autocorrelation methods.

The preferred version of the algorithm comprises the following steps:

- Detection of the non-EIG signals. The signal may include non-EIG segments, such as artifacts and silences. The signal is scanned and the non-EIG segments are marked and ignored.

  - The signal is divided into overlapping analysis windows, each four seconds long and each has a 75% overlap with the adjacent windows. The analysis window contains more than one event.
  - A modified form of an Autocorrelation transform of the type used for pitch detection in speech processing is applied to a single analysis window. This step is repeated for each analysis window.
  - The fundamental frequency is derived from the autocorrelation values of the analysis window. Usually the fundamental frequency is indicated by the largest autocorrelation value. This step is repeated for each analysis window.
  - A postprocessor is used to "smooth out" the results of all the individual analysis windows. The previously marked non-EIG segments ( artifacts and silences ) are added in this phase.

To produce the modified autocorrelation transform, the existing autocorrelation based algorithm was adapted to EIG in the following ways:

- An improved algorithm was devised for determining pitch from the autocorrelation values. The algorithm usually chooses the highest autocorrelation peak ( value ). In EIG we found that sometimes the true pitch is not represented by a peak, but rather by a valley between several adjacent peaks. An algorithm was devised to locate those cases, and to correctly estimate the pitch. We refer to this phenomena as a "volcano" shaped autocorrelation graph, because the center of the "mountain" is found on lower ground.

- A voiced/unvoiced decision mechanism was adapted from speech processing. The "unvoiced" EIG segments were defined as a non-signals (undecided segments). A postprocessor was used to decide on the pitch of those undecided segments. Although unvoiced speech segments do exist, "unvoiced" EIG segments are a virtual non-signal, and do not really exist.

- A special pre-processing algorithm was devised. The signal undergoes convolution so as to increase the width of the event. This is unlike speech pre-processing, which is aimed at enhancing the high amplitude portions and/or filtering out the formants. This preprocessing technique is referred to as "fattening".

- A very long analysis window of 4 seconds was used, in order to find frequencies ranging from 0.25 Hz to 5 Hz. In speech it is customary to use a window of about 30 milliseconds, in order to find frequencies ranging from 80 Hz to 300 Hz.

The invention also contemplates the use of a Segmented Autocorrelation algorithm. This is considered to be the best method for EIG, but other methods are also adequate. Segmented autocorrelation is described in "Pitch detection of speech signals using Segmented Autocorrelation"/ I. A. Atkinson, A. M. Kondoz, B.G. Evans/ Electronics Letters Vol. 31 No. 7 pp. 533-535/March 1995.

Brief Description of the Drawings

The foregoing brief description, as well as further objects, features, and advantages of the present invention will be understood more completely from the following detailed description of a presently preferred embodiment, with reference being had to the accompanying drawings, in which:

FIG. 1 is a functional block diagram showing an overview of the process of the preferred embodiment;
FIG. 2 is a flowchart describing the pre-detectors, the silence detector and the artifact detector,
FIG. 3 is a flowchart describing fundamental frequency estimation of an individual analysis window;
FIG. 4 is a flowchart expansion of one of the block 25 of FIG 3 and describes the decision algorithm of the fundamental frequency estimator, and
FIG. 5 is a flowchart describing the post-processing phase algorithms, which combine information from neighboring analysis windows in order to correct local estimation errors.

Detailed Description of the Preferred Embodiment

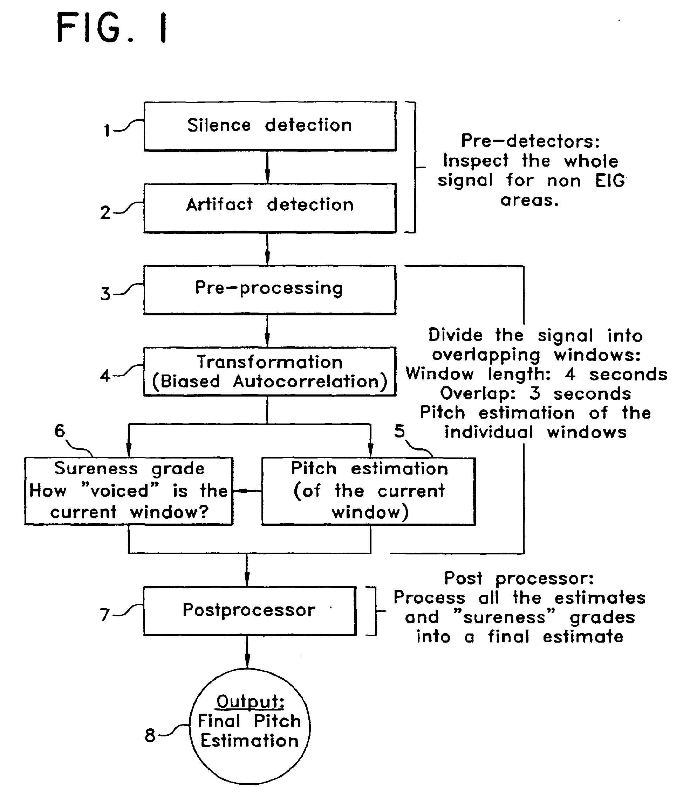

The preferred embodiment of the invention will now be described in detail with reference to the accompanying drawings. First a schematic overview of the preferred embodiment of a process and system for fundamental frequency estimation of EIG is presented in **FIG. 1**. The algorithm comprises of the following steps:

a. Detection of the non-EIG signals. The signal may include non-EIG segments, such as artifacts and silences. The signal is scanned and the non-EIG segments are marked and ignored. A silence detector **1** and an artifact detector **2** are used. An "artifact" is a dominant interference noise signal, which severely corrupts the EIG signal.

b. The signal is divided into overlapping analysis windows. Each window is 4 seconds long and has a 75% overlap with the adjacent windows. The analysis window must contain more than one event. It is contemplated that the analysis window could be as much as forty times the interval between successive events.

c. The autocorrelation transform **4** is applied to a single analysis window. This step is repeated for each analysis window. pre-processing **3** is optional, and is not used in the preferred embodiment.

d. The fundamental frequency is derived from the autocorrelation values of the analysis window **5**. Usually the fundamental frequency is indicated by the largest autocorrelation value. A sureness measure **6** is computed for the estimated fundamental frequency of the analysis window. This step is repeated for each analysis window.

e. A postprocessor **7** is used to "smooth out" the results of all the individual analysis windows. The previously marked non-EIG segments ( artifacts and silences ) are added in this phase.

f. The output is the estimated fundamental frequency of the signal **8**.

The preferred embodiment of the invention includes software which preferably runs on a Pentium PC, using the Windows 95 operating system. The algorithm was implemented on the "Matlab" software by "The Mathworks Inc." The algorithm was written in the Matlab language, and runs within the Matlab program shell. It also requires the "Digital Signal Processing Toolbox" for Matlab, by "The Mathworks Inc."

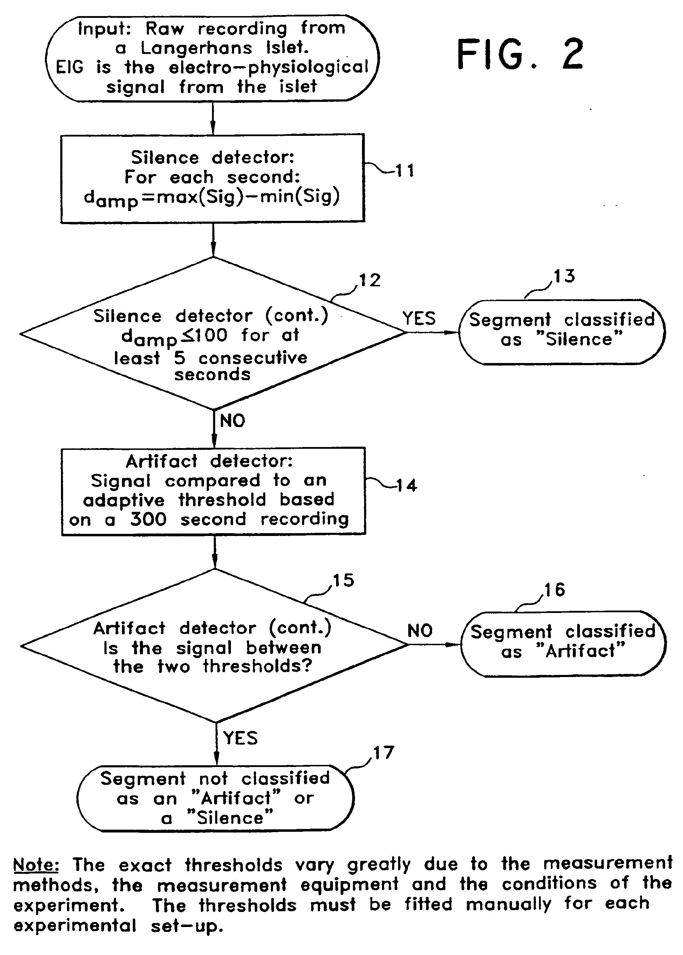

FIG 2. is a flowchart describing the pre-detectors. The silence detector ( 11, 12 ,13 ) involves two steps. In the first step 11 an amplitude related measure is computed for each second, and in the second step 12 the amplitude related measure is compared to a fixed threshold. "Sig" is the raw digital input signal. It is the raw signal recorded from the islet, after an Analog to Digital conversion. In block 11 each one second window is examined. "Max(Sig)" is the maximum sample value within the examined one second window. "Min(Sig)" is the minimum of the samples' values within the examined one second window. As "Sig" is always a real number, the real maximum and minimum are computed. If the measure is below the threshold for more than 5 seconds, then the segment is classified as silence 13. The threshold depends on the measurement equipment and environment. It also depends on the digitizing scale. In the preferred embodiment the maximum amplitude of the. EIG signal is approximately 4000 amplitude units, so a threshold of 100 amplitude units is used. An "amplitude unit" is the amplitude difference between two adjacent quantization levels of the Analog to Digital converter. In the preferred embodiment one "amplitude unit" corresponds to 0.25 Micro-Volt of the original electrical signal. "Original" means before amplification.

The artifact detector ( 14, 15, 16, 17 ) involves two steps. In the first step 14 the signal is compared to an adaptive threshold. The adaptive threshold is computed using a 300 second long recording, by the following computation:

A high reference is defined as the 99.833 percentile of the histogram of the amplitude values of the 300 second long recording.

A low reference is defined as the 0.167 percentile of the histogram of the amplitude values of the 300 second long recording.

An amplitude reference is defined as the high reference minus the low reference.

A high threshold is defined as the high reference plus the amplitude reference.

A low threshold is defined as the low reference minus the amplitude reference.

The "histogram" used in block 14 is generated by simply sorting all the sample values ("amplitudes") of a 300 second long recording. The sorting is done from the smallest value to the largest value. Based on those sorted values the two references are derived. The "high reference" is the 99.833 percentile of the sorted values. The "low reference" is the 0.167 percentile of the sorted values. The term "histogram" refers to the sorting of the values, and can be replaced by the term "sorted amplitude values."

The second step 15 involves checking whether there are samples in which the signal's amplitude is above the high threshold or below the low threshold. If such samples are found then they are classified as containing an artifact 16. The remaining samples 17 are not classified as an artifact or as a silence.

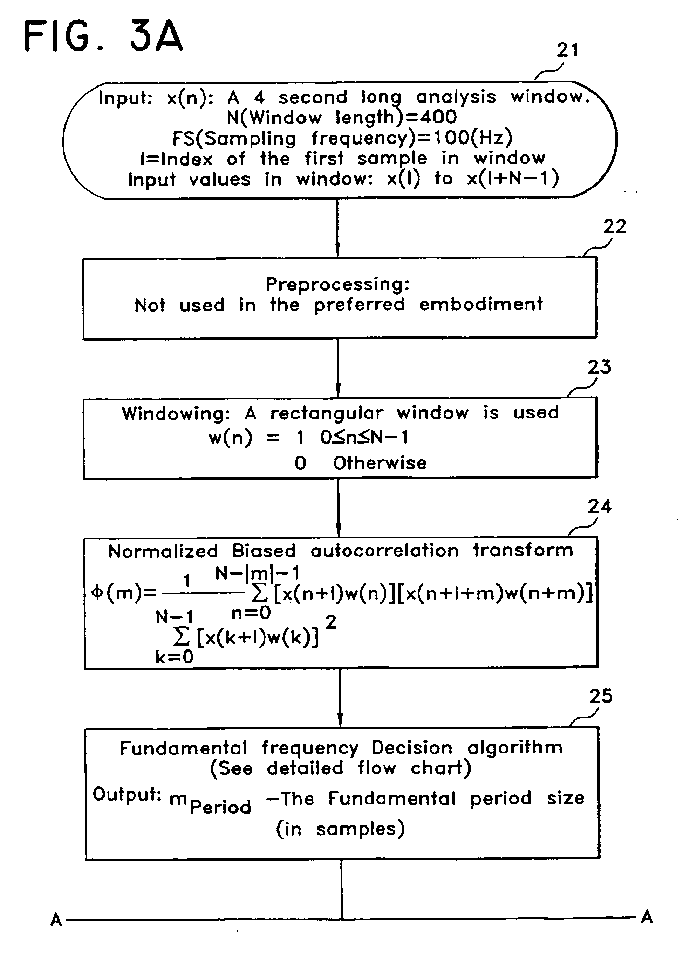

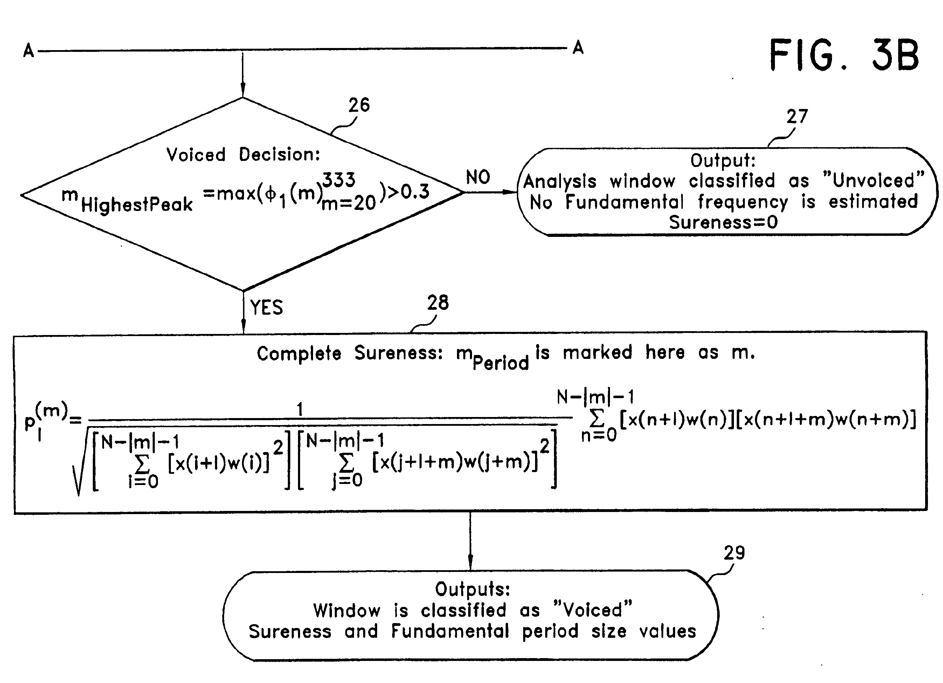

FIG. 3 contains a flowchart describing the steps that are performed on each analysis window. Each analysis window is 4 seconds long. Overlapping analysis windows are used. There is a 3 second overlap between two successive windows. Analysis windows containing either silences or artifacts are not analyzed. The analysis windows are described in block 21, and are represented mathematically by the following notation:

$x(n)$ are the raw digital samples of the signal. We earlier referred to this raw input signal as "Sig." This is the same signal, only that here a more strict mathematical notation is used. The analysis window contains 4 seconds of signal. The sampling frequency ("FS") in the preferred embodiment is 100 Hz, therefore the analysis window contains 400 samples. This is marked by "N(Window length)=400." The first sample in the analysis window is $x(l)$, where "$l$" is the index of the sample. Therefore the last sample in the analysis window is $x(li+399)=x(l+N-1)$. Therefore the analysis window is described by the samples it contains from $x(l)$ to $x(l+N-l)$.

Preprocessing 22 may be used, but it is not implemented in the preferred embodiment.

A rectangular window 23 is applied to the samples, and a normalized biased autocorrelation transform 24 is computed. It is represented mathematically by the following equation:

$$\varphi(m)=\frac{1}{\sum_{k=0}^{N-1}[x(k+1)w(k)]^2}\sum_{n=0}^{N-|m|-1}[x(n+1)w(n)][x(n+l+m)w(n+m)] \qquad (1)$$

where "w(n)" is the windowing function." As "w(n)" is a rectangular window, as described in block 23, it is equivalent to "1" within the analysis window:

$$w(n)= \begin{matrix} 1 & 0 \leq n \leq N-1 \\ 0 & Otherwise \end{matrix}$$

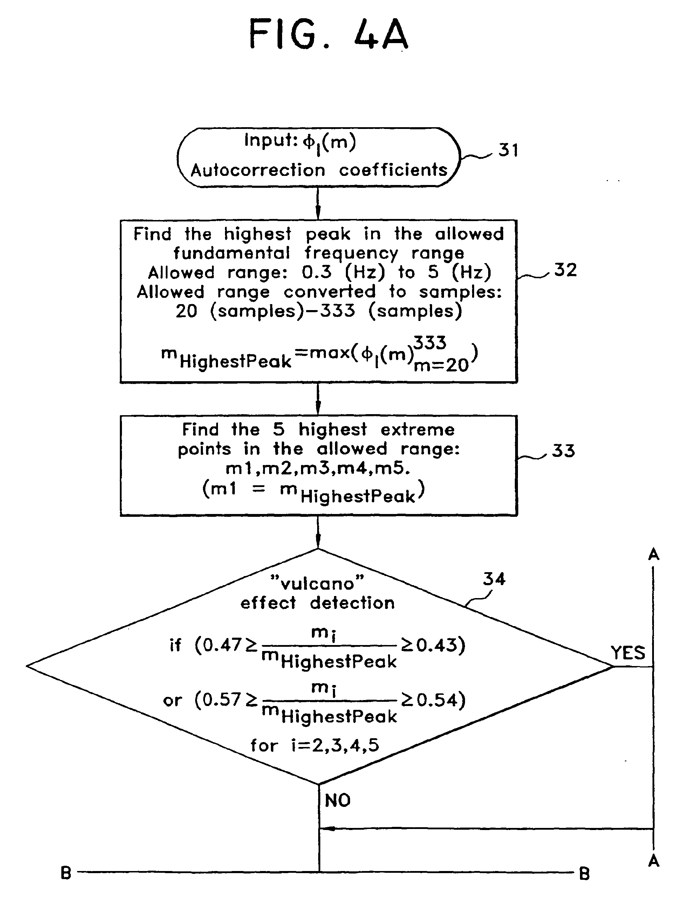

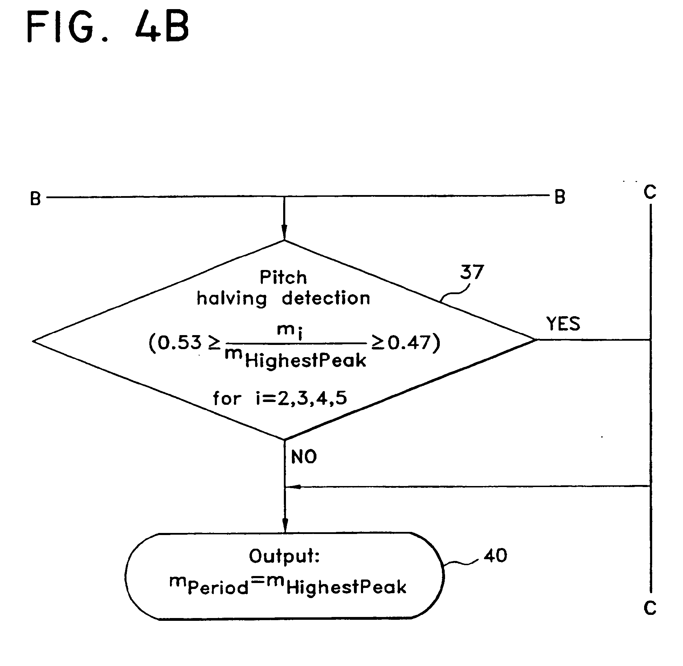

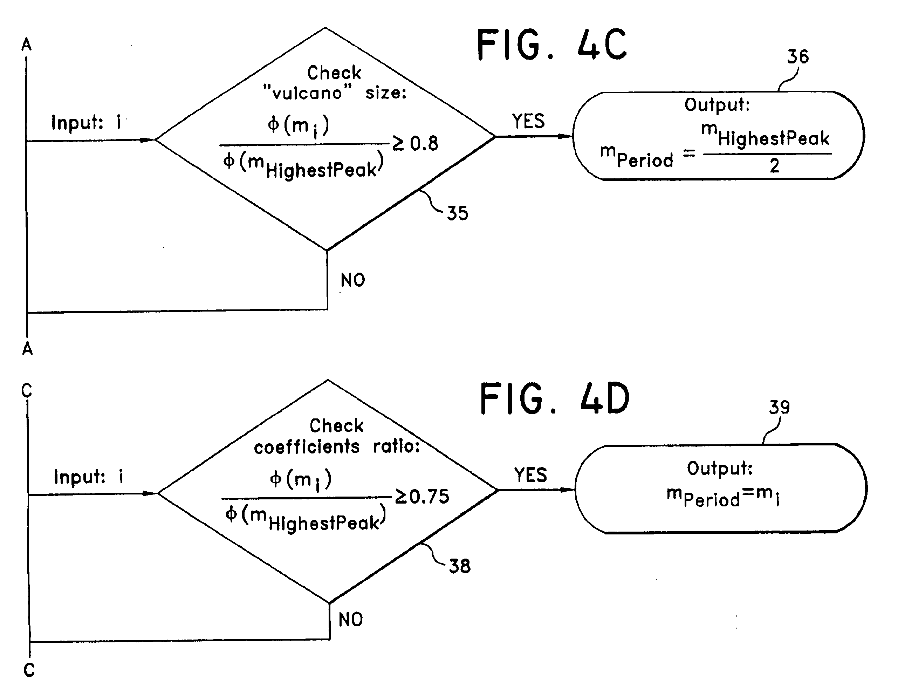

The autocorrelation transform is further described in the book "Digital Processing of Speech Signals", L.R. Rabiner and R.W. Schafer, 1978, pp. 141-164. The fundamental frequency decision algorithm **25** is described in more detail in **FIG. 4**.

We will now refer to **FIG. 4** and later return to **FIG. 3**.

**FIG. 4** is a flowchart describing the fundamental frequency decision algorithm. Actually it decides on the fundamental period, from which the fundamental frequency can be derived. An improved algorithm was devised for determining the fundamental frequency from the autocorrelation values. In typical algorithms the highest autocorrelation peak (value) is usually chosen. In EIG, it was found that sometimes the true pitch is not represented by a peak, but rather by a valley between several adjacent peaks. An algorithm was devised to locate those cases, and to estimate the pitch correctly. We refer to this phenomenon as a "volcano" shaped autocorrelation graph, because the center of the "mountain" is found on lower ground.

The input to the decision algorithm is a vector containing the autocorrelation coefficients **31**. Steps **32** and **33** find the 5 highest extreme points within the allowed frequency range. A "volcano" effect detector **34** checks first whether one of the highest peaks is found in a suspected "volcano" area. If such a peak is found, it's index *(i)* is passed on in order to check the peak's amplitude **35** compared to the amplitude of the highest peak. If the result is positive then a "volcano" is detected **36** and the estimated fundamental period is half of the autocorrelation lag corresponding to the highest peak. The autocorrelation is represented by equation (1) above. *m* is the "autocorrelation lag." It is the lag between the two segments on which the correlation is computed. The first segment is *x(n+l)* to *x(n+l+N-|m|-1)* and the second segment is *x(n+l+m)* to *(x+l+m+N-|m|-1)*. Also, see "Digital Processing of Speech Signals", L.R. Rabiner and R.W. Schafer, 1978, pp. 141-164.

A pitch halving detection algorithm **37** first checks the ratio of the autocorrelation coefficients' lag **37**, and then their amplitudes **38**. If one of the highest peaks fulfills both those conditions then it is chosen as the estimated fundamental period **39**. If both the "volcano" detector and the pitch halving detector produced negative results, then the estimated fundamental period is assumed to be the lag of the highest autocorrelation peak **40**. The output of the fundamental period decision algorithm of **FIG. 4** is the estimated fundamental period. This output is returned to block 26 in **FIG. 3**.

A "voiced" decision mechanism **26** (**Fig. 3**) is used to decide whether the fundamental frequency should be estimated in the current analysis window. If the maximum autocorrelation coefficient in the allowed lag range ( a lag of 20 to 333 samples ) is below 0.3, then the analysis window is classified as "unvoiced", and no fundamental frequency is estimated **27**. In that case a sureness grade of zero is given to the analysis window.

The voiced / unvoiced decision mechanism was adapted from speech processing. The "unvoiced" EIG segments were defined as non-signals ( undecided segments). The postprocessor will be used in later stages to decide on the fundamental frequency of those undecided segments. Note that unvoiced speech segments do exist, while "unvoiced" EIG segments are a virtual non-signal, and do not really exist.

If the current analysis window is not classified as "unvoiced" then the estimation process continues, and a sureness grade is calculated for the current analysis window **28**. A higher sureness grade indicates that there is a higher probability that the fundamental frequency was estimated correctly. For each analysis window several outputs 29 are returned:

1. The estimated fundamental period- this value is not returned if the analysis window is classified as "unvoiced";
2. A "voiced"/"unvoiced" decision- specifies whether the fundamental period was estimated in the analysis window; and
3. A sureness grade- this value is not returned if the analysis window is classified as "unvoiced".

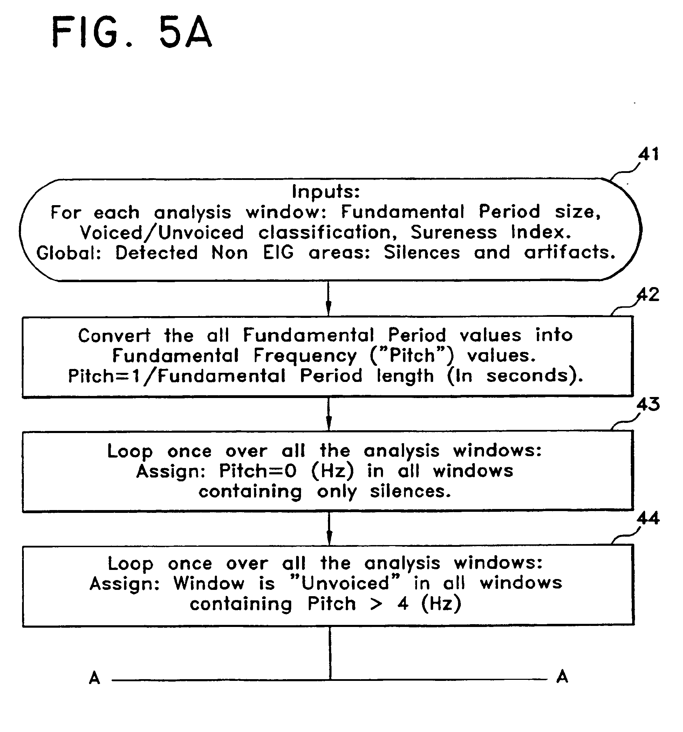

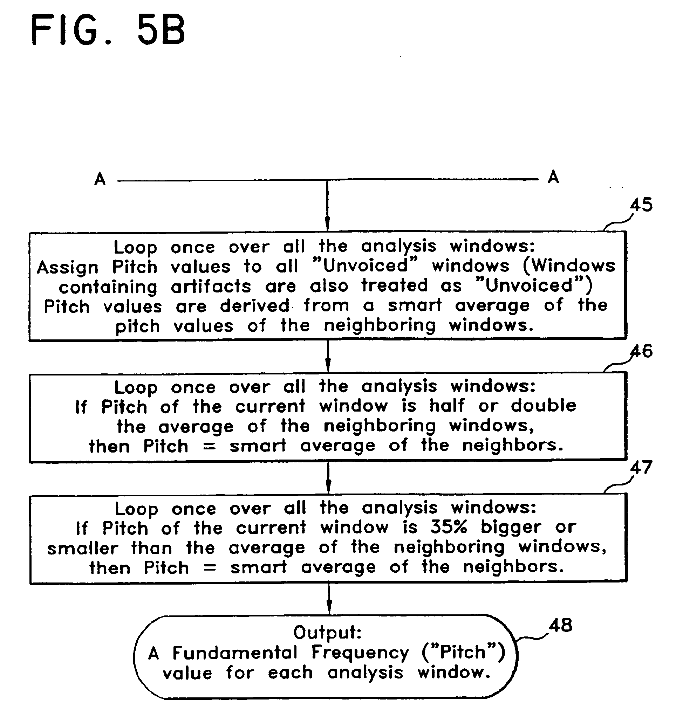

**FIG. 5** describes the final stage of the estimation process: the postprocessor. The postprocessor combines all the acquired data **41** into a fundamental frequency estimate of the raw signal. The postprocessor operates on a 300 second long recording of the raw signal. The first step **42** is to convert all the estimated fundamental period values into estimated fundamental frequency ( "Pitch" ) values. The output of this stage is a fundamental frequency estimate, in Hertz, for every "voiced" analysis window. The next step **43** inserts the previously detected silence segments 1 into the fundamental frequency estimation results. The fundamental frequency is assigned a value of 0 ( Hz ) in all windows containing only silences. All analysis windows containing an unlikely fundamental frequency estimate are marked as "unvoiced" in the next step **44**.

The next step **45** assigns a fundamental frequency ( "pitch" ) value to the "unvoiced" segments. The postprocessor loops once over all the analysis windows. It assigns Pitch values to all "unvoiced" windows ( windows containing artifacts are also treated as "unvoiced" ). Pitch values are derived from an average of the pitch values of the neighboring windows. Some minor data dependent modifications can be added in order to create a smarter averaging method, but those are minor changes which are very data dependent. A pitch halving / doubling error correcting mechanism is then applied **46**. The algorithm loops once over all the analysis windows. If the Pitch of the current window is half or double the average of the neighboring windows, then the current Pitch is taken as a smart average of the neighbors. The term "smart averaging" refers to the average of an ensemble of numbers, after the extreme values are removed. For example: We can define a "smart average" of 8 numbers in the following way:

1. Remove the maximal and minimal numbers and
2. Compute the average of the remaining 6 numbers. This "smart averaging" method is used in order to achieve more stable and robust results.

The final step **47** looks for an analysis window having a pitch which is significantly different than its neighbors. The algorithm loops once over all the analysis windows. If the Pitch of the current window, is 35% greater or smaller than the average of the neighboring windows, then the current Pitch is taken as a smart average of the neighbors.

The final output **48** of the entire fundamental frequency estimation process is a fundamental frequency value for each analysis window.

Although a preferred embodiment of the invention has been disclosed for illustrative purposes, those skilled in the art will appreciate that many additions, modifications and substitutions are possible without departing from the scope and spirit of the invention. For example, the principles and applications of the invention are not limited to the particular biological phenomenon described. They could be utilized equally well, for example, for measuring an optical signal from living cells of a muscle or the electrical signals of neurons, or various signals produced by similar or other biological micro-structures. In particular, it is contemplated that the invention could find utility in any application where a cell or other biological micro-structure is moved outside its natural environment and used as a biological sensor.

**Claims**

1. A system for monitoring biological activity of living cells, which activity produces detectable signals characterizing events within the cells, comprising a sensor capable of receiving the sensible signals from the cells and a processor including a module for estimating the fundamental frequency of the occurrence of events from the detectable signals, **characterized in that** the processor estimates the fundamental frequency of occurrence of events without first detecting the occurrence of individual events, that the module produces an analysis window during which events are analysed, the analysis window spanning more than one event, that the module for estimating includes components to perform an autocorrelation operation, the components being configured to perform the autocorrelation operation by locating and estimating a pitch that is represented by a valley between adjacent autocorrelation peaks.

2. The system of claim 1 wherein the module for estimating further includes one of the following submodules;
   a submodule which estimates the fundamental frequency based upon a lower autocorrelation value disposed among several adjacent peaks;
   a submodule which identifies "unvoiced" segments of the detectable signal as undecided as to pitch, the pitch of those segments being estimated by a subsequent processing submodule; be
   a submodule which controls the analysis window to be in the range of .25 to 5 Hertz;
   a submodule controlling the analysis window to have a duration in the range of several seconds; and
   a submodule performing a pre-processing operation which has the effect of increasing the effective duration of an event.

3. The system of any of claims 1 or 2 wherein the processor is constructed to perform a segmented autocorrelation process.

4. The system of any of claims 1 to 3 wherein the sensor is a probe capable of detecting signals emitted by living cells in the Islets of Langerhans, the frequency estimate being an indication of blood glucose level of a patient in which those cells are present.

5. The system of any preceding claim wherein an analysis window spans a duration of up to 40 times the interval between successive events.

6. A method for monitoring biological activity of living cells, which activity produces detectable signals characterizing events within the cells, **characterized in that** the fundamental frequency of the occurrence of events is estimated from the detectable signals, without detecting the occurrence of individual events, wherein the events are signals produced by the Islets of Langerhans.

7. The method of claim 6 wherein events are analysed during an analysis window which spans more than one event.

8. The method of any of claims 6 or 7 wherein the events are signals produced by biological micro-structures which are displaced from their original environment.

9. The method of any preceding claim wherein the estimating step includes an autocorrelation operation.

10. The method of claim 9 further including one of the following steps:

   estimating the fundamental frequency based upon a lower autocorrelation value disposed among several adjacent peaks;
   treating "unvoiced" segments of the detectable signal as undecided as to pitch and estimating the pitch of those segments through subsequent processing;
   seeking to estimate the fundamental frequency in the range of 25 to 5 Hertz;
   utilizing an analysis window duration in the range of several seconds;
   performing a pre-processing operation which has the effect of increasing the effective duration of an event; and
   utilizing an autocorrelation process which performs segmented autocorrelation.

11. The method of any of claims 6 to 10 further comprising the step of using the number of "unvoiced" windows within a predetermined time as a measure of a blood constituent level of a patient.

12. The method of any of claims 6 to 10 further comprising the step of using a sureness grade as measure of a blood or tissue constituent level of a patient.

13. The method of any of claims 6 to 10 further comprising the step of using the fundamental frequency as a measure of a blood or tissue constituent level of a patient.

14. The method of claim 13 wherein the blood constituent level is the blood glucose level in the vicinity of the biological micro-structure.

15. The method of any of claims 6 to 10 wherein the events are electrical signals produced by living cells in the Islets of Laugerhans used as a probe within a patient and the fundamental frequency estimate is used as a measure of blood glucose level of the patient.

16. The method of claim 7 wherein an analysis window spans a duration of up to 40 times the interval between successive events.

**Patentansprüche**

1. System zur Überwachung der biologischen Aktivität lebender Zellen, welche Aktivität nachweisbare Signale produziert, die Ereignisse in den Zellen kennzeichnen, umfassend einen Sensor, der zum Empfang der sensiblen Signale aus den Zellen fähig ist und einen Prozessor, der ein Modul zur Bestimmung der Grundfrequenz des Auftretens von Ereignissen aus den nachweisbaren Signalen einschließt, **dadurch gekennzeichnet, dass** der Prozessor die Grundfrequenz des Auftretens von Ereignissen bestimmt, ohne zuerst das Auftreten von individuellen Ereignissen nachzuweisen, dass das Modul ein Analysenfenster produziert, während welchem Ereignisse analysiert werden, wobei das Analysenfenster mehr als ein Ereignis überspannt, dass das Modul zur Bestimmung Komponenten zur Durchführung eines Autokorrelationsvorgangs einschließt, wobei die Komponenten zur Durchführung des Autokorrelationsvorgangs durch Lokalisierung und Bestimung eines Abstands, der durch einen Talbereich benachbarter Autokorrelationspeaks dargestellt ist, konfiguriert werden.

2. System nach Anspruch 1, wobei das Modul zur Bestimmung weiter eines der folgenden Submodule einschließt;
ein Submodul, das die Grundfrequenz bezogen auf einen niedrigeren Autokorrelationswert bestimmt, der unter mehreren benachbarten Peaks verteilt ist;
ein Submodul, das "stimmlose" Segmente des nachweisbaren Signals als unentschieden in Bezug auf den Abstand identifiziert, wobei der Abstand von diesen Segmenten durch ein sich anschließendes Verarbeitungssubmodul bestimmt wird;
ein Submodul, welches das Analysenfenster kontrolliert, das im Bereich von 0,25 bis 5 Hertz sein soll;
ein Submodul, welches das Analysenfenster kontrolliert, das eine Dauer im Bereich von mehreren Sekunden aufweisen soll; und
ein Submodul, das einen Vorgang vor der Verarbeitung durchführt, der den Effekt der Verlängerung der wirksamen Dauer eines Ereignisses aufweist.

3. System nach einem der Ansprüche 1 oder 2, wobei der Prozessor zur Durchführung eines segmentierten Autokorrelationsvorgangs konstruiert ist.

4. System nach einem der Ansprüche 1 bis 3, wobei der Sensor eine Sonde darstellt, die zum Nachweis von durch lebende Zellen in den Langerhans-Inseln abgegebenen Signalen fähig ist, wobei die Bestimmung der Frequenz einen Hinweis auf den Blutglukosespiegel eines Patienten, bei dem diese Zellen vorliegen, darstellt.

5. System nach einem der vorangehenden Ansprüche, wobei ein Analysenfenster eine Dauer von bis zum 40fachen des Intervalls zwischen aufeinanderfolgenden Ereignissen überspannt.

6. Verfahren zum Überwachen der biologischen Aktivität lebender Zellen, welche Aktivität nachweisbare Signale produziert, die Ereignisse in den Zellen kennzeichnen, **dadurch gekennzeichnet, dass** die Grundfrequenz des Auftretens von Ereignissen aus den nachweisbaren Signalen, ohne den Nachweis des Auftretens individueller Ereignisse, bestimmt wird, wobei die Ereignisse Signale darstellen, die von den Langerhans-Inseln produziert werden.

7. Verfahren nach Anspruch 6, wobei Ereignisse während eines Analysenfensters, das mehr als ein Ereignis überspannt, analysiert werden.

8. Verfahren nach einem der Ansprüche 6 oder 7, wobei die Ereignisse Signale darstellen, die von biologischen Mikrostrukturen, die aus ihrer ursprünglichen Umgebung verdrängt sind, produziert werden.

9. Verfahren nach einem der vorangehenden Ansprüche, wobei der Bestimmungsschritt einen Autokorrelationsvorgang einschließt.

10. Verfahren nach Anspruch 9, das weiter einen der folgenden Schritte einschließt:

Bestimmung der Grundfrequenz bezogen auf einen unteren Autokorrelationswert, der unter mehreren benachbarten Peaks verteilt ist;
Behandlung "stimmloser" Segmente des nachweisbaren Signals als unentschieden in Bezug auf den Abstand und Bestimmung des Abstands der Segmente durch die sich anschließende Verarbeitung;
Versuch der Bestimmung der Grundfrequenz im Bereich von 0,25 bis 5 Hertz;
Nutzung einer Analysenfenster-Dauer im Bereich von mehreren Sekunden;
Durchführung eines Vorgangs vor der Verarbeitung, der den Effekt zur Verlängerung der wirksamen Dauer eines Ereignisses aufweist; und
Nutzung eines Autokorrelationsvorgangs, der eine segmentierte Autokorrelation durchführt.

11. Verfahren nach einem der Ansprüche 6 bis 10, das weiter den Schritt der Verwendung der Anzahl "stimmloser" Fenster in einer prädeterminierten Zeit als ein Maß einer Konzentration der Blutbestandteile von einem Patienten umfasst.

12. Verfahren nach einem der Ansprüche 6 bis 10, das weiter den Schritt der Verwendung eines Sicherheitsgrads als ein Maß einer Konzentration der Blut- oder Gewebebestandteile von einem Patienten umfasst.

13. Verfahren nach einem der Ansprüche 6 bis 10, das weiter den Schritt der Verwendung der Grundfrequenz als ein Maß einer Konzentration der Blut- oder Gewebebestandteile von einem Patienten umfasst.

14. Verfahren nach Anspruch 13, wobei die Konzentration der Blutbestandteile den Blutglukosespiegel in der Umgebung der biologischen Mikrostruktur darstellt.

15. Verfahren nach einem der Ansprüche 6 bis 10, wobei die Ereignisse elektrische Signale darstellen, die von lebenden Zellen in den Langeihans-Inseln produziert werden, die als eine Sonde in einem Patienten verwendet werden und die Bestimmung der Grundfrequenz als ein Maß des Blutglukosespiegels des Patienten verwendet wird.

16. Verfahren nach Anspruch 7, wobei ein Analysenfenster eine Dauer von bis zum 40fachen des Intervalls zwischen aufeinanderfolgenden Ereignissen überspannt.


## Revendications

1. Système pour contrôler l'activité biologique des cellules vivantes, laquelle activité produit des signaux détectables caractérisant des événements dans les cellules, comprenant un détecteur capable de recevoir les signaux sensibles provenant des cellules et un processeur incluant un module pour estimer la fréquence fondamentale de la survenue des événements à partir des signaux détectables, **caractérisé en ce que** le processeur estime la fréquence fondamentale de la survenue des événements sans détecter tout d'abord la survenue des événements individuels, que le module produit une fenêtre d'analyse durant laquelle les événements sont analysés, la fenêtre d'analyse couvrant plus d'un événement, que le module pour estimer inclut des composants pour effectuer une opération d'autocorrélation, les composants étant configurés de manière à effectuer l'opération d'autocorrélation en localisant et en estimant un niveau qui est représenté par une vallée entre des pics d'autocorrélation adjacents.

2. Système selon la revendication 1, dans lequel le module pour estimer comprend, en outre, l'un des sous-modules suivants;
un sous-module qui estime la fréquence fondamentale ayant pour base une valeur d'autocorrélation inférieure disposée parmi plusieurs pics adjacents ;
un sous-module qui identifie les segments «muets» du signal détectable comme étant incertains quant au niveau, le niveau de ces segments étant estimé par un sous-module de traitement ultérieur ;
un sous-module qui contrôle la fenêtre d'analyse pour être entre 0,25 et 5 Hertz ;
un sous-module contrôlant la fenêtre d'analyse pour avoir une durée d'une plage de plusieurs secondes ; et
un sous-module effectuant une opération de prétraitement qui a l'effet de prolonger la durée effective d'un événement.

3. Système selon la revendication 1 ou la revendication 2, dans lequel le processeur est construit pour effectuer un procédé d'autocorrélation segmenté.

4. Système selon l'une quelconque des revendications 1 à 3, dans lequel le détecteur est une sonde capable de détecter des signaux émis par des cellules vivantes dans les îlots de Langerhans, l'estimation de la fréquence étant une indication du taux de glycémie d'un patient chez qui ces cellules sont présentes.

5. Système selon l'une quelconque des revendications précédentes, dans lequel une fenêtre d'analyse couvre une durée équivalant au maximum à 40 fois l'intervalle entre des événements successifs.

6. Méthode de contrôle de l'activité biologique des cellules vivantes, laquelle activité produit des signaux détectables caractérisant des événements dans les cellules, **caractérisée en ce que** la fréquence fondamentale de la survenue des événements est estimée à partir des signaux détectables, sans détecter la survenue des événements individuels, dans laquelle les événements sont des signaux produits par les îlots de Langerhans.

7. Méthode selon la revendication 6, dans laquelle les événements sont analysés durant une fenêtre d'analyse qui couvre plus d'un événement.

8. Méthode selon la revendication 6 ou la revendication 7, dans laquelle les événements sont des signaux produits par des microstructures biologiques qui sont déplacées par rapport à leur milieu d'origine.

**9.** Méthode selon l'une quelconque des revendications précédentes, dans laquelle l'étape d'estimation comprend une opération d'autocorrélation.

**10.** Méthode selon la revendication 9 incluant, en outre, l'une des étapes suivantes consistant à :

estimer la fréquence fondamentale ayant pour base une valeur d'autocorrélation inférieure disposée parmi plusieurs pics adjacents;
traiter des segments « muets » du signal détectable comme étant incertains quant au niveau et estimer le niveau de ces segments par traitement ultérieur ;
chercher à estimer la fréquence fondamentale comprise entre 0,25 et 5 Hertz ;
utiliser une durée de fenêtre d'analyse dans la plage de plusieurs secondes ;
effectuer une opération de prétraitement qui a l'effet de prolonger la durée effective d'un événement ; et
utiliser un procédé d'autocorrélation qui effectue une autocorrélation segmentée.

**11.** Méthode selon l'une quelconque des revendications 6 à 10 comprenant, en outre, l'étape utilisant le nombre de fenêtres « muettes » dans un délai prédéterminé en tant que mesure du taux d'un constituant sanguin d'un patient.

**12.** Méthode selon l'une quelconque des revendications 6 à 10 comprenant, en outre, l'étape utilisant un degré de sûreté en tant que mesure du taux d'un constituant sanguin ou tissulaire d'un patient.

**13.** Méthode selon l'une quelconque des revendications 6 à 10 comprenant, en outre, l'étape utilisant la fréquence fondamentale en tant que mesure du taux d'un constituant sanguin ou tissulaire d'un patient.

**14.** Méthode selon la revendication 13, dans laquelle le taux de constituant sanguin est le taux de glycémie à proximité de la microstructure biologique.

**15.** Méthode selon l'une quelconque des revendications 6 à 10, dans laquelle les événements sont des signaux électriques produits par des cellules vivantes dans les îlots de Langerhans utilisés en tant que sonde dans un patient et l'estimation de la fréquence fondamentale est utilisée en tant que mesure du taux de glycémie du patient.

**16.** Méthode selon la revendication 7, dans laquelle une fenêtre d'analyse couvre une durée équivalant au maximum à 40 fois l'intervalle entre des événements successifs.

# FIG. I

**Silence detection** — 1

**Artifact detection** — 2

Pre-detectors:
Inspect the whole signal for non EIG areas.

**Pre-processing** — 3

**Transformation (Biased Autocorrelation)** — 4

Divide the signal into overlapping windows:
Window length: 4 seconds
Overlap: 3 seconds
Pitch estimation of the individual windows

6 — **Sureness grade How "voiced" is the current window?**

5 — **Pitch estimation (of the current window)**

**Postprocessor** — 7

Post processor:
Process all the estimates and "sureness" grades into a final estimate

8 — **Output: Final Pitch Estimation**

# FIG. 2

Input: Raw recording from a Langerhans Islet. EIG is the electro-physiological signal from the islet

Silence detector:
For each second:
$d_{amp} = max(Sig) - min(Sig)$ — 11

Silence detector (cont.)
$d_{amp} \leq 100$ for at least 5 consecutive seconds — 12

→ YES → Segment classified as "Silence" — 13

↓ NO

Artifact detector:
Signal compared to an adaptive threshold based on a 300 second recording — 14

Artifact detector (cont.)
Is the signal between the two thresholds? — 15

→ NO → Segment classified as "Artifact" — 16

↓ YES

Segment not classified as an "Artifact" or a "Silence" — 17

<u>Note:</u> The exact thresholds vary greatly due to the measurement methods, the measurement equipment and the conditions of the experiment. The thresholds must be fitted manually for each experimental set-up.

# FIG. 3A

**21**

Input: x(n): A 4 second long analysis window.
N(Window length)=400
FS(Sampling frequency)=100(Hz)
I=Index of the first sample in window
Input values in window: x(I) to x(I+N−1)

**22**

Preprocessing:
Not used in the preferred embodiment

**23**

Windowing: A rectangular window is used
$$w(n) = 1 \quad 0 \leq n \leq N-1$$
$$0 \quad \text{Otherwise}$$

**24**

Normalized Biased autocorrelation transform

$$\phi(m) = \frac{1}{N-1} \frac{\sum_{n=0}^{N-|m|-1} [x(n+I)w(n)][x(n+I+m)w(n+m)]}{\sum_{k=0}^{N-1} [x(k+I)w(k)]^2}$$

**25**

Fundamental frequency Decision algorithm
(See detailed flow chart)
Output: $m_{Period}$ −The Fundamental period size
(in samples)

A ——————————————————————————— A

FIG. 3B

A ——————————————————————————— A

**26**

Voiced Decision:

$$m_{HighestPeak} = \max(\phi_1(m)_{m=20}^{333}) > 0.3$$

**NO** →

**27**

Output:
Analysis window classified as "Unvoiced"
No Fundamental frequency is estimated
Sureness=0

**YES** ↓

**28**

Complete Sureness: $m_{Period}$ is marked here as m.

$$p_l^{(m)} = \frac{1}{\sqrt{\left[\sum_{i=0}^{N-|m|-1}[x(i+l)w(i)]^2\right]\left[\sum_{j=0}^{N-|m|-1}[x(j+l+m)w(j+m)]^2\right]}}\sum_{n=0}^{N-|m|-1}[x(n+l)w(n)][x(n+l+m)w(n+m)]$$

**29**

Outputs:
Window is classified as "Voiced"
Sureness and Fundamental period size values

EP 1 082 051 B1

# FIG. 4A

Input: $\phi_I(m)$
Autocorrection coefficients — 31

Find the highest peak in the allowed
fundamental frequency range
Allowed range: 0.3 (Hz) to 5 (Hz)
Allowed range converted to samples:
20 (samples)—333 (samples)

$$m_{HighestPeak} = max(\phi_I(m)_{m=20}^{333})$$

— 32

Find the 5 highest extreme
points in the allowed range:
m1,m2,m3,m4,m5.
$(m1 = m_{HighestPeak})$

— 33

"vulcano"
effect detection                              34

if $(0.47 \geq \dfrac{m_i}{m_{HighestPeak}} \geq 0.43)$

or $(0.57 \geq \dfrac{m_i}{m_{HighestPeak}} \geq 0.54)$

for i=2,3,4,5

YES

NO

A

A

B —————————————————————— B

15

# FIG. 4B

B ─────────────────────────────────── B · · C

Pitch
halving detection

$$\left(0.53 \geq \frac{m_i}{m_{HighestPeak}} \geq 0.47\right)$$

for i=2,3,4,5

37

YES

NO

Output:
$m_{Period} = m_{HighestPeak}$

40

C

## FIG. 4C

A

Input: i → Check "vulcano" size:

$$\frac{\phi(m_i)}{\phi(m_{HighestPeak})} \geq 0.8$$

— 35

YES → Output:

$$m_{Period} = \frac{m_{HighestPeak}}{2}$$

36

NO

A

## FIG. 4D

C

Input: i → Check coefficients ratio:

$$\frac{\phi(m_i)}{\phi(m_{HighestPeak})} \geq 0.75$$

— 38

YES → Output:

$$m_{Period} = m_i$$

39

NO

C

EP 1 082 051 B1

# FIG. 5A

**41**

Inputs:
For each analysis window: Fundamental Period size,
Voiced/Unvoiced classification, Sureness Index.
Global: Detected Non EIG areas: Silences and artifacts.

**42**

Convert the all Fundamental Period values into
Fundamental Frequency ("Pitch") values.
Pitch=1/Fundamental Period length (In seconds).

**43**

Loop once over all the analysis windows:
Assign: Pitch=0 (Hz) in all windows
containing only silences.

**44**

Loop once over all the analysis windows:
Assign: Window is "Unvoiced" in all windows
containing Pitch > 4 (Hz)

A ———————————————————————— A

# FIG. 5B

A ———————————————————— A

45

Loop once over all the analysis windows:
Assign Pitch values to all "Unvoiced" windows (Windows
containing artifacts are also treated as "Unvoiced")
Pitch values are derived from a smart average of the
pitch values of the neighboring windows.

46

Loop once over all the analysis windows:
If Pitch of the current window is half or double
the average of the neighboring windows,
then Pitch = smart average of the neighbors.

47

Loop once over all the analysis windows:
If Pitch of the current window is 35% bigger or
smaller than the average of the neighboring windows,
then Pitch = smart average of the neighbors.

48

Output:
A Fundamental Frequency ("Pitch")
value for each analysis window.